(19) Europäisches Patentamt
European Patent Office
Office européen des brevets

(11) Numéro de publication : **0 552 999 A1**

(12) # DEMANDE DE BREVET EUROPEEN

(21) Numéro de dépôt : **93400051.4**

(22) Date de dépôt : **13.01.93**

(51) Int. Cl.$^5$ : **C07K 7/06,** C07K 7/08, A61K 37/02

Le demandeur a ultérieurement déposé une liste des séquences et déclaré, que cette liste ne comporte aucun élément nouveau.

(30) Priorité : **15.01.92 FR 9200340**

(43) Date de publication de la demande :
**28.07.93 Bulletin 93/30**

(84) Etats contractants désignés :
**AT BE CH DE DK ES FR GB GR IE IT LI LU NL PT SE**

(71) Demandeur : **ADIR ET COMPAGNIE**
**1 rue Carle Hébert**
**F-92415 Courbevoie Cédex (FR)**

(72) Inventeur : **Fauchere, Jean-Luc**
**13 rue d'Orléans**
**F-92210 Sait-Cloud (FR)**
Inventeur : **Thurieau, Christophe**
**120 rue de Silly**
**F-92100 Boulogne sur Seine (FR)**
Inventeur : **Verbeuren, Tony**
**60 bis rue Aristide Braind**
**F-78540 Vernouillet (FR)**
Inventeur : **Paladino, Joseph**
**4 rue des Coudriers**
**F-78700 Conflans Sainte Honorine (FR)**

(54) **Dérivés de l'hirudine.**

(57) L'invention concerne les dérivés de formule (I) :

$$X - A_1 - A_2 - A_3 - A_4 - A_5 - A_5 - A_7 - A_5 - A_5 - A_{10} - Y \qquad (I)$$

dans laquelle X, $A_1$, $A_2$, $A_3$, $A_4$, $A_5$, $A_5$, $A_7$, $A_5$, $A_9$, $A_{10}$ et Y sont tels que définis dans la description.
Médicaments.

EP 0 552 999 A1

La présente invention concerne de nouveaux dérivés de peptides et de pseudopeptides therapeutiquement actifs dans la cascade de coagulation sanguine, leur procédé de préparation et les compositions pharmaceutiques qui les contiennent.

Il est désormais largement connu que lorsque l'équilibre entre facteurs procoagulants et anticoagulants dans le sang est perturbé, il peut en résulter la formation d'un thrombus ou caillot de sang. Le développement d'une thrombose est favorisé essentiellement par trois facteurs pathogéniques principaux qui sont la stase ou diminution du flux sanguin, les états d'hypercoagulabilité et les lésions de l'endothélium de la paroi vasculaire. Contre ces pathogénies, il convient donc d'établir un traitement dont l'une des bases principales est le médicament anticoagulant.

Les anticoagulants sont en effet utilisables dans le traitement des thromboses veineuses aigües, de l'embolie pulmonaire, de l'embolie artérielle des extrêmités, des thromboses artérielles comme l'infarctus du myocarde et de toutes les autres manifestations thromboemboliques.

Parmi les agents anticoagulants connus, l'hirudine, qui est un polypeptide comportant 65 acides aminés, est un inhibiteur spécifique de la thrombine isolé des glandes salivaires de la sangsue médicinale (Biochemistry 25, 4622-28, 1986).

Des variantes de l'hirudine utilisables en tant qu'inhibiteur de thrombine ont déjà été décrits. C'est le cas, par exemple, des composés décrits dans les brevets EP 209061 ou EP 332523. D'autre part, des analogues synthétiques de fragments d'hirudine à propriétés anticoagulantes ont également été décrits ; c'est le cas, par exemple, des composés revendiqués dans les brevets EP 276014, EP 291981, EP 291982 et EP 333356. Par rapport au modèle naturel, ces fragments plus courts (10 à 20 acides aminés) offrent l'avantage d'être plus "maniables" : en particulier leur synthèse est plus simple.

Plus récemment, la demande de brevet européen EP 0372503 a revendiqué des peptides analogues de l'hirudine dans lesquels un acide aminé naturel était remplacé par un acide aminé synthétique.

La demande EP 0443598 revendique des peptides analogues de l'hirudine dans lesquelles un acide naturel est remplacé par un dérivé sulfoné ou phosphoné.

Les demandes PCT 91/01328 et EP 443429 revendiquent des analogues de l'hirudine dans lesquels les modifications portent à la fois sur l'introduction d'amino-acides non naturels mais également sur l'introduction de sulfono-oxo ou phosphono-oxo-amino-acides.

Il est vrai qu'un peptide est une structure fragile, facilement hydrolysable et les modifications apportées par l'art antérieur ont pour but d'augmenter la stabilité de l'analogue synthétisé.

Tous les dérivés revendiqués par l'art antérieur sont par ailleurs caractérisés par une formule générale qui est $X-A_A-A_B-A_C-A_D-A_E-A_F-A_G-A_H-A_I-A_J-Y$ où le radical X substituant le résidu aminé terminal est soit un atome d'hydrogène soit un ou deux groupements alkyles de 1 à 6 atomes de carbone, ou un ou deux groupements acylés de 2 à 10 atomes de carbone, ou un groupement benzyloxycarbonyl ou t.butyloxycarbonyle.

La demanderesse a présentement découvert des composés pour lesquels la valeur du radical X a été sensiblement modifiée. Les dérivés obtenus ont une activité antithrombotique, anticoagulante et/ou antiagrégante plaquettaire nettement supérieure à celle des dérivés les plus proches de l'art antérieur.

Ainsi, les composés de l'invention ont une activité anticoagulante au minimum 30 fois supérieure à celle du fragment d'hirudine 55-65 soit une activité au moins trois fois supérieure à celle des composés les plus proches de l'art antérieur.

Cette puissance d'activité jointe à leur haute stabilité rend les composés de l'invention particulièrement intéressants pour un usage thérapeutique.

L'invention concerne plus particulièrement de nouveaux dérivés peptidiques répondant à la formule générale (I) :

$$X - A_1 - A_2 - A_3 - A_4 - A_5 - A_6 - A_7 - A_8 - A_9 - A_{10} - Y \qquad (I)$$

dans laquelle :

X représente le substituant du groupement aminé terminal du peptide de formule (I) et représente un groupement guanidinoacyl ($C_1$-$C_6$) linéaire ou ramifié choisi parmi les groupements

$X_O$ = 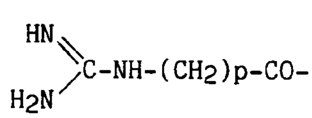 ,

avec $1 \leqq p \leqq 5$ ,

un groupement guanidinoalkyl ($C_1$-$C_6$) linéaire ou ramifié ou l'un quelconque des groupements suivants :

$X_1$ = 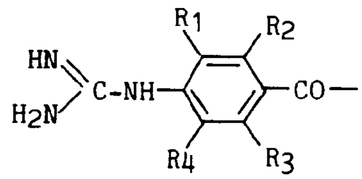

avec $R_1$, $R_2$, $R_3$, $R_4$ identiques ou différents représentant indépendamment l'un de l'autre un atome d'hydrogène, un groupement alkyle ($C_1$-$C_6$) linéaire ou ramifié, un atome d'halogène, un groupement trifluorométhyle, un groupement hydroxy, un groupement alkoxy ($C_1$-$C_6$) linéaire ou ramifié, un groupement thio, un groupement alkylthio ($C_1$-$C_6$) linéaire ou ramifié, un groupement cyano, un groupement nitro ou un groupement phényloxy,

$X_2$ = 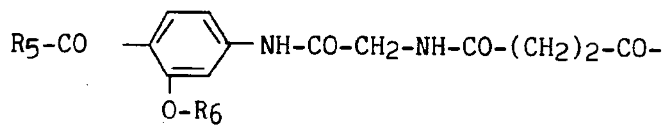 ,

où $R_5$ représente un groupement alkoxy inférieur ($C_1$-$C_6$) ou un groupement hydroxyle et où $R_5$ représente un atome d'hydrogène ou un groupement acyle inférieur ($C_1$-$C_6$),
ou

$X_3$ = 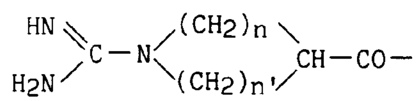 ,

avec n et n' entiers compris entre 1 et 6,

$A_1$ représente une liaison ou un résidu peptidique contenant de 1 à 3 amino-acides quelconques, parmi lesquels peut figurer un résidu $A_{11}$ de formule :

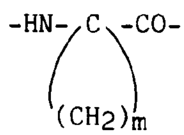

(avec m entier compris entre 2 et 7)

$A_2$ représente une liaison ou un résidu phénylalanine (Phe), tyrosine (Tyr), isoleucine (Ile), norvaline (Nva), (1,2,3,4)-tétrahydro-isoquinoline-3-carbonyl (Tic), un résidu acide α-aminobutyrique (Abu), acide glutamique (Glu), ou un résidu d'acide aminé contenant un groupement aryle, un résidu $A_{11}$ ou un résidu $A_9$,

$A_3$ représente une liaison ou un résidu acide glutamique (Glu), acide aspartique (Asp), β-alanine (βAla), tyrosine (Tyr) ou un résidu $A_{11}$,

$A_4$ représente une liaison ou un résidu acide glutamique (Glu), acide aspartique (Asp), proline (Pro), acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh) ou, un résidu $A_{11}$,

$A_5$ représente une liaison ou un résidu isoleucine (Ile), norvaline (Nva), phénylalanine (Phe), proline (Pro), ornithine (Orn), acide 2,3-diaminopropionique (Dpr) ou un résidu $A_{11}$,

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro), un résidu $A_{11}$ ou l'acide pipécolique (Pip),

$A_7$ représente un résidu acide glutamique (Glu), acide aspartique (Asp) ou un résidu $A_{11}$,

$A_5$ représente un résidu acide glutamique (Glu), acide aspartique (Asp), acide 2,3-diaminopropionique (Dpr), acide 2,4-diaminobutyrique (Dab), β-alanine (βAla) ou un résidu $A_{11}$,

$A_9$ représente un résidu

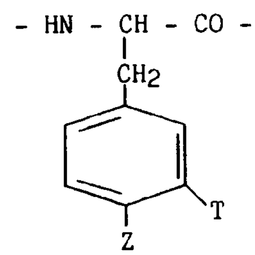

avec Z ou T, différents, représentant un atome d'hydrogène ou un groupement $OPO_3H_2$, $PO_3H_2$, $CH_2CO_2H$ ou OH à la condition que lorsque Z représente OH, T soit différent d'un atome d'hydrogène,

$A_{10}$ représente une liaison, un résidu leucine (Leu), valine (Val), β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), un résidu tripeptidique tel que Nal-Nal-Leu ou un résidu $A_{11}$ ou un fragment peptidique contenant de 1 à 3 résidus de tout acide aminé,

Y représente le substituant du groupement carbonyl terminal du peptide de formule (I) et représente un groupement hydroxy, un groupement alkoxy ($C_1$-$C_6$) linéaire ou ramifié ou un groupement amino lui-même éventuellement substitué par un ou deux groupements alkyles ($C_1$-$C_6$) linéaires ou ramifiés,
ou bien
le groupement carboxy terminal du peptide de formule (I) est réduit en alcool correspondant (ol), (groupement $-CH_2OH$),

étant entendu que par groupement aryle on entend phényle ou naphtyle,

le composé de formule I pouvant éventuellement comprendre :

- une liaison pseudopeptidique telle que $-CH_2$-NH-, $-CH_2$-S-, $-CH_2$-SO-, $-CH_2-SO_2$-, -NH-CO-, -CH=CH- remplaçant une liaison peptidique -CO-NH-,
et/ou
- une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,

leurs sels d'addition à un acide ou à une base pharmaceutiquement acceptable, chaque amino-acide de la séquence peptidique étant optiquement pur et le carbone a de chaque amino-acide ayant la configuration D ou L.

Parmi les acides pharmaceutiquement acceptables on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables on peut citer à titre non limitatif l'hydroxyde de sodium, l'hydroxyde de potassium, la triéthylamine, la tertbutylamine, etc...

L'expression "tout acide aminé" utilisée par la présente demande inclut aussi bien les acides aminés naturels que les acides aminés dits non protéiques communément utilisés par l'homme de métier dans la synthèse d'analogues synthétiques de peptides naturels.

Parmi les acides aminés naturels, on peut citer, à titre d'exemple et de façon non limitative, la glycine, l'alanine, la valine, la leucine, l'isoleucine, la sérine, la méthionine, la thréonine, la phénylalanine, la tyrosine, le tryptophanne, la cystéine, la proline, l'histidine, l'acide aspartique, l'asparagine, la glutamine, l'acide glutamique, l'arginine, l'ornithine et la lysine.

Parmi les acides aminés non protéiques, on peut citer, à titre d'exemple et de façon non limitative, la norleucine, la norvaline, l'alloisoleucine, l'homoarginine, la thiaproline, la déhydroproline, l'hydroxyproline, l'homosérine, la cyclohexylglycine, la phénylglycine, l'acide amino n-butyrique, la cyclohexylalanine, l'acide aminophénylbutyrique, les phénylalanines mono, di ou tri substituées sur le groupement phényl en position ortho, meta ou para par le(s) groupement(s) choisi(s) parmi : ($C_1$-$C_6$) alkyle, ($C_1$-$C_6$) alkoxy, halogène, trifluorométhyle, nitro, amino, mono ou di ($C_1$-$C_6$) alkyl amino ou encore un groupement méthylène dioxy, β-2 et 3-thiénylalanine, β-2 et 3-furylalanine, β-2, 3 et 4-pyridylalanine, β-(2 et 3-benzothiényl)alanine, β-(1- et 2-naphtyl)alanine, des dérivés O-alkylés de la sérine, thréonine, tyrosine, les cystéines S-alkylées, tous les acides aminés de configuration D, isomères des acides aminés naturels de configuration L, etc.

L'invention s'étend aussi au procédé de préparation des dérivés de formule (I) qui peuvent être obtenus par différentes méthodes telles que la synthèse séquentielle sur phase solide, la synthèse de fragments et leur couplage en solution, la synthèse enzymatique, la synthèse génétique par clonage et expression de gènes dans des bactéries transformées ou par des combinaisons diverses de ces techniques.

Les méthodes générales de la synthèse des peptides sur phase solide ont été décrites par B.W. ERICKSON et R.B. MERRIFIELD ("The Proteins", Solid-Phase Peptide Synthesis, 3ème édition, 257-527, 1976).

La synthèse sur phase solide peut se réaliser sur un automate qui effectue de façon répétitive et programmable des cycles de déprotection, couplage et lavages nécessaires à l'introduction séquentielle des acides aminés dans la chaîne peptidique. L'acide aminé, de préférence C-terminal est fixé sur une résine conventionnellement utilisée pour la préparation de polypeptides, de préférence un polystyrène réticulé à l'aide de 0,5 à 3,0 % de divinylbenzène et muni de restes activés tels que le chlorométhylène ou l'hydroxyméthylène qui permettent la fixation covalente du premier acide aminé sur la résine. Le choix approprié de la résine permet la fixation d'une fonction C-terminale acide carboxylique, amide ou alcool.

Les acides aminés sont alors introduits un à un dans l'ordre déterminé par l'opérateur. Chaque cycle de synthèse correspondant à l'introduction d'un acide aminé, comporte une déprotection, de préférence N-terminale de la chaîne peptidique, des lavages successifs destinés à éliminer les réactifs ou à gonfler la résine, un couplage avec activation de l'acide aminé et de nouveaux lavages. Chacune de ces opérations est suivie d'une filtration réalisée grâce à la présence d'un verre fritté incorporé au réacteur dans lequel s'effectue la synthèse.

Les réactifs de couplage utilisés sont des réactifs classiques de la synthèse peptidique comme le dicyclohexylcarbodiimide (DCC) et l'hydroxybenzotriazole (HOBT) ou le benzotriazol-1-yl-oxytris (diméthylamino) phosphonium hexafluorophosphate (BOP) ou encore le diphenylphosphorylazide (DPPA).

Des activations par formation d'anhydrides mixtes sont également possibles.

Chaque acide aminé est introduit dans le réacteur en quadruple excès environ, par rapport au degré de substitution de la résine et en quantité environ équivalente par rapport aux agents de couplage. La réaction de couplage peut être vérifiée à chaque étape de la synthèse par le test de réaction à la ninhydrine décrit par E. KAISER et Coll. (Analyt. Biochem., 34, 595, 1970).

Après assemblage de la chaîne peptidique sur la résine, un traitement par un acide fort tel que l'acide trifluoroacétique, ou l'acide fluorhydrique en présence d'anisole, d'éthanedithiol ou de 2-méthylindole sert à séparer le peptide de la résine ainsi qu'à libérer éventuellement le peptide de ses groupes protecteurs. Le composé est alors purifié par des techniques classiques de purification, notamment chromatographiques.

La cyclisation totale ou partielle des composés de l'invention est réalisée en solution de préférence à travers deux fonctions déprotégées de façon sélective et capables de former une liaison covalente comme une liaison amide.

Les peptides de la présente invention peuvent être également obtenus par couplage en solution de fragments peptidiques sélectivement protégés, qui peuvent être préparés soit sur phase solide, soit en solution. L'emploi des groupes protecteurs et la mise à profit de leur stabilité différentielle est analogue aux méthodes sur phase solide à l'exception de l'attachement de la chaîne peptidique sur la résine. Le groupe carboxylique C-terminal est protégé, par exemple, par un ester méthylique ou une fonction amide. Les méthodes d'activation lors des couplages sont également analogues à celles employées dans la synthèse sur phase solide.

La synthèse de peptides contenant des liaisons pseudopeptidiques telles que $-CH_2-NH-$, $-CH_2-S-$, $-CH_2-SO-$, $-CH_2-SO_2-$, -NH-CO-, -CH=CH- est effectuée soit par les méthodes en solution soit de façon combinée avec la synthèse sur phase solide en utilisant les méthodes classiques de la chimie organique. Ainsi, par exemple, l'introduction de la liaison $-CH_2-NH$ se fait en préparant en solution l'aldéhyde Fmoc-NH-CHR-CHO selon la technique décrite par FEHRENTZ et CASTRO (Synthesis, 676-678, 1983) et en le condensant avec la chaîne peptidique croissante soit sur phase solide selon la technique décrite par SASAKI et COY (Peptides, 8, 119-121, 1988), soit en solution.

Les composés de formule (I) possèdent des propriétés pharmacologiques très intéressantes.

Ils sont doués de propriétés anticoagulantes et antithrombotiques et peuvent ainsi être utilisés pour prévenir les complications post-thromboemboliques par dissolution des caillots ou en tant qu'agents préventifs de l'extension du processus thrombotique en les utilisant comme anticoagulants d'action directe et rapide. Leurs propriétés d'inhibition de la voie d'activation plaquettaire médiée par la thrombine permettent d'envisager leur utilisation en tant qu'inhibiteur des étapes de l'interaction des plaquettes sanguines avec la paroi vasculaire impliquées dans les phénomènes de thrombose et d'athérosclérose.

La présente invention a également pour objet les compositions pharmaceutiques renfermant comme principe actif au moins un composé de formule générale (I) ou un de ses sels d'addition à un acide ou à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols, ampoules buvables, injectables, etc...

La posologie varie selon l'âge et le poids du patient, la nature et la sévérité de l'affection ainsi que la voie d'administration.

Celle-ci peut être orale, nasale, rectale ou parentérale. D'une manière générale, elle s'échelonne entre 0,05 et 30 mg pour un traitement en une ou plusieurs prises par 24 heures.

Les exemples suivants illustrent l'invention et ne la limitent en aucune façon.

Dans les exemples ci-dessous, les acides aminés dont les abréviations commencent par une lettre majuscule sont de configuration L. Les acides aminés dont les abréviations commencent par une lettre minuscule sont de configuration D.

L'acide aminé nommé Abo est de configuration 3S.

L'acide aminé nommé Oic est de configuration 2S,3aS,7aS.

Le symbole G représente le radical 4-guanidinobenzoyle :

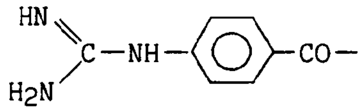

Tyr ($mPO_3H_2$) représente le résidu :

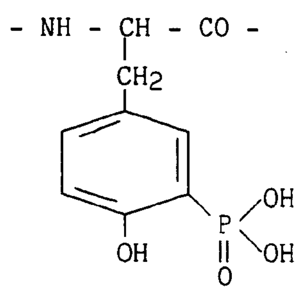

Tyr ($pPO_3H_2$) représente le résidu :

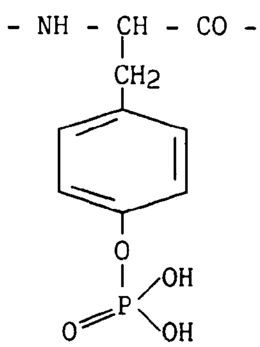

Phe ($pPO_3H_2$) représente le résidu :

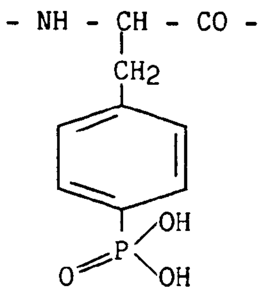

Phe ($pCH_2CO_2H$) représente le résidu :

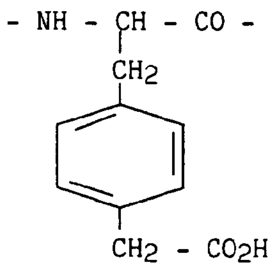

**EXEMPLE 1 : G-Gly-Asp-Phe-Glu-Abo-Ile-Pro-Glu-Glu-Tyr($mPO_3H_2$)-Leu-glu-OH, trifluoroacétate**

Le composé de l'exemple 1 est synthétisé à partir de 2 g d'une résine substituée par 0,33 mmole/g de Fmoc-glu(OtBu)-OH et suivant le protocole répétitif suivant :

| N° opération | Fonction | Solvant/Réactif | Répétition/temps |
|---|---|---|---|
| 1 | lavage | DMF | 2 × 2 min |
| 2 | déprotection | 20 % pipéridine/DMF | 1 × 5 min |
| 3 | déprotection | 20 % pipéridine/DMF | 1 × 15 min |
| 4 | lavage | DMF | 3 × 2 min |
| 5 | lavage | dichlorométhane | 3 × 2 min |
| 6 | couplage | acide aminé protégé activé | 1 × 90 min |
| 7 | lavage | DMF | 3 × 2 min |
| 8 | lavage | isopropylique alcool | 3 × 2 min |
| 9 | lavage | dichlorométhane | 3 × 2 min |

Chacune de ces opérations effectuée dans 30 ml de solvant, sous agitation et à température ambiante, est suivie d'une filtration à travers un verre fritté incorporé à la cellule de verre (réacteur) dans laquelle la synthèse progresse. Le filtre retient la résine sur laquelle est fixée la chaîne peptidique croissante.

Les acides aminés protégés choisis ont été introduits dans l'ordre suivant : Fmoc-Leu-OH, Fmoc-Tyr[(pOBzl),($mPO_3(CH_3)_2$)]-OH, Fmoc-glu(OtBu)-OH,
Fmoc-glu(OtBu)-OH, Fmoc-Pro-OH, Fmoc-Ile-OH, Fmoc-Abo-OH,
Fmoc-glu(OtBu)-OH, Fmoc-Phe-OH, Fmoc-Asp(OtBu)-OH, Fmoc-Gly-OH ;

L'activation en vue du couplage (opération 6) est obtenue à chaque cycle par dissolution de 4 équivalents (2.64 mmoles) de l'acide aminé protégé avec 360 mg d'HOBt dans 30 ml de DMF, puis après 30 minutes à température ambiante, par addition de 618 mg de DCC. Cette solution est alors immédiatement introduite dans la cellule de réaction avec 10 ml de dichlorométhane.

A la fin des neuf cycles correspondant à la fixation séquentielle de onze acides aminés, on a ainsi obtenu

avec l'acide glutamique C-terminal, un dodécapeptide protégé sur ses chaînes latérales et fixé sur la résine. La condensation de la chaine peptidique avec l'acide 4-guanidinobenzoïque est effectuée en présence de TBTU, HOBT, DIEA. Celle-ci est alors traitée par un mélange d'acide trifluoroacétique (18 ml), éthanedithiol (1 ml) et anisole (1 ml) pendant 90 minutes à température ambiante. Le filtrat et les solvants de lavage de la résine sont réunis et évaporés à sec. Le produit est précipité dans l'éther, filtré et séché puis purifié par CLHP préparative, sur colonne $C_{18}$ (diamètre interne : 47 mm, longueur : 300 mm) et lyophilisé.

L'analyse du produit obtenu est réalisé après décomposition de celui-ci en acides aminés par hydrolyse dans de l'acide chlorhydrique 6N pendant 18 heures à 110°C et dosage quantitatif des acides aminés obtenus par CLHP.

| | Asp | Gly | Glu | Leu | Phe | Pro | Tyr | Ile + Abo |
|---|---|---|---|---|---|---|---|---|
| calculé | 1 | 1 | 4 | 1 | 1 | 1 | 1 | 2 |
| trouvé | 0,97 | 1,10 | 3,78 | 1,00 | 1,05 | 1,03 | 1,10 | 1,94 |

Spectre de masse (FAB) : $MH^+$, $m/_z$ = 1718

Les exemples suivants ont été préparés en utilisant le mode opératoire décrit dans l'exemple 1.

**EXEMPLE 2 : G-Acc-Asp-Phe-Glu-Abo-Ile-Pro-Glu-Glu-Tyr($pPO_3H_2$)-Leu-glu-OH, trifluoroacétate**

La résine substituée par Fmoc-Leu-glu(OtBu)-OH est condensée en présence de pipéridine de la même façon que dans l'exemple 1 avec Fmoc-Tyr[$OPO_3(tBu)_2$]-OH ; cet intermédiaire est obtenu à partir de Fmoc-Tyr-OH traité en trois étapes :

a/ Silylation carboxyle utilisant le chlorure de tert-butyldiméthyle silyle en présence de N-méthylmorpholine.

b/ Phosphorylation utilisant $(Et)_2NP(OtBu)_2$ en présence de 1H tétrazole.

c/ Oxydation, déprotection utilisant une solution aqueuse d'hydroperoxyde de t-butyle et de bisulfite de sodium ($Na_2S_2O_5$).

Les intermédiaires ne sont pas isolés et le produit final Fmoc-Tyr[$OPO_3(tBu)_2$]-OH est purifié classiquement avant la condensation. En fin de synthèse, l'acide paraguanidinobenzoïque est condensé avec la chaine peptidique préalablement obtenue en présence de BOP, HOBT et N-méthylmorpholine.

**EXEMPLE 3 : G-Gly-Asp-Phe-Glu-Abo-Ile-Pro-Glu-Glu-Tyr($pPO_3H_2$)-Leu-glu-OH, trifluoroacétate**

Analyse :

| | Asp | Phe | Glu | Abo + Leu + Ile | Pro | Gly | Tyr |
|---|---|---|---|---|---|---|---|
| calculé | 1 | 1 | 4 | 3 | 1 | 1 | 1 |
| trouvé | 1,03 | 1,05 | 4,01 | 2,80 | 1,01 | 1,05 | 1,05 |

Spectre de masse (FAB) : $MH^+$, $m/_z$ = 1718

**EXEMPLE 4** : **G-Acc-Asp-Phe-Glu-Abo-Ile-Pip-Glu-Glu-Tyr($mPO_3H_2$)-Leu-glu-OH, trifluoroacétate**

**EXEMPLE 5** : **G-Acc-Asp-Phe-Glu-Abo-Ile-Pip-Glu-Glu-Tyr($pPO_3H_2$)-Leu-glu-OH, trifluoroacétate**

**EXEMPLE 6** : **G-Gly-Asp-Tyr($pPO_3H_2$)-Glu-Abo-Ile-Pro-Glu-Glu-Tyr($pPO_3H_2$)-Leu-glu-OH, trifluoroacétate**

**EXEMPLE 7** : **G-Gly-Asp-Phe-Glu-Abo-Ile-Oic-Glu-Glu-Tyr($pPO_3H_2$)-Leu-glu-OH, trifluoroacétate**

**EXEMPLE 8** : **G-Acc-Asp-Phe-Glu-Abo-Ile-Oic-Glu-Glu-Tyr($pPO_3H_2$)-Leu-glu-OH, trifluoroacétate**

**EXEMPLE 9** : **G-Acc-Asp-Tyr($pPO_3H_2$)-Glu-Abo-Ile-Pro-Glu-Glu-Tyr($pPO_3H_2$)-Leu-glu-OH, trifluoroacétate**

**EXEMPLE 10** : **G-Acc-Asp-Tyr($pPO_3H_2$)-Glu-Abo-Ile-Oic-Glu-Glu-Tyr($pPO_3H_2$)-Leu-glu-OH, trifluoroacétate**

**EXEMPLE 11** : **G-Gly-Asp-Tyr($pPO_3H_2$)-Glu-Abo-Ile-Oic-Glu-Glu-Tyr($pPO_3H_2$)-Leu-glu-OH, trifluoroacétate**

**EXEMPLE 12** : **G-Gly-Asp-Tyr($pPO_3H_2$)-Glu-Abo-Ile-Pro-Glu-Glu-Tyr($mPO_3H_2$)-Leu-glu-OH, trifluoroacétate**

**EXEMPLE 13** : **G-Gly-Asp-Tyr($pPO_3H_2$)-Glu-Abo-Ile-Oic-Glu-Glu-Tyr($mPO_3H_2$)-Leu-glu-OH, trifluoroacétate**

**EXEMPLE 14** : **G-Acc-Asp-Tyr($pPO_3H_2$)-Glu-Abo-Ile-Pro-Glu-Glu-Tyr($mPO_3H_2$)-Leu-glu-OH, trifluoroacétate**

**EXEMPLE 15** : **G-Acc-Asp-Phe-Glu-Abo-Ile-Pro-Glu-Glu-Phe($pPO_3H_2$)-Leu-glu-OH, trifluoroacétate**

**EXEMPLE 16** : **G-Gly-Asp-Phe-Glu-Abo-Ile-Oic-Glu-Glu-Phe($pPO_3H_2$)-Leu-glu-OH, trifluoroacétate**

**EXEMPLE 17** : **G-Gly-Asp-Phe-Glu-Abo-Ile-Pro-Glu-Glu-Phe($pPO_3H_2$)-Leu-glu-OH, trifluoroacétate**

Microanalyse élémentaire :

| | Asp | Glu | Gly | Pro | Phe | Ile + Abo | Leu |
|---|---|---|---|---|---|---|---|
| calculé | 1 | 4 | 1 | 1 | 1 | 2 | 1 |
| trouvé | 0,98 | 3,92 | 1,02 | 0,99 | 1,00 | 2,02 | 0,97 |

Spectre de masse (FAB) : $MH^+$, m/z = 1702

**<u>EXEMPLE 18</u> : G-Acc-Asp-Phe-Glu-Abo-Ile-Oic-Glu-Glu-Phe($pPO_3H_2$)-Leu-glu-OH, trifluoroacétate**

**<u>EXEMPLE 19</u> : G-Acc-Asp-Phe($pPO_3H_2$)-Glu-Abo-Ile-Pro-Glu-Glu-Phe($pPO_3H_2$)-Leu-glu-OH, trifluoroacétate**

**<u>EXEMPLE 20</u> : G-Acc-Asp-Phe($pPO_3H_2$)-Glu-Abo-Ile-Oic-Glu-Glu-Phe($pPO_3H_2$)-Leu-glu-OH, trifluoroacétate**

**<u>EXEMPLE 21</u> : G-Gly-Asp-Phe($pPO_3H_2$)-Glu-Abo-Ile-Oic-Glu-Glu-Phe($pPO_3H_2$)-Leu-glu-OH, trifluoroacétate**

**<u>EXEMPLE 22</u> :$X_0$-Gly-Asp-Tyr-Glu-Abo-Ile-Pro-Glu-Glu-Tyr($pPO_3H_2$)-Leu-glu-OH, trifluoroacétate**

avec pour $X_0$ : p = 1

**<u>EXEMPLE 23</u> : $X_0$-Gly-Asp-Tyr-Glu-Abo-Ile-Oic-Glu-Glu-Tyr($pPO_3H_2$)-Leu-glu-OH, trifluoroacétate**

avec pour $X_0$ : p = 1

**<u>EXEMPLE 24</u> : $X_0$-Acc-Asp-Tyr-Glu-Abo-Ile-Pro-Glu-Glu-Tyr($pPO_3H_2$)-Leu-glu-OH, trifluoroacétate**

avec pour $X_0$ : p = 1

**<u>EXEMPLE 25</u>** : **$X_2$-Gly-Asp-Phe-Glu-Abo-Ile-Pip-Glu-Glu-Tyr($mPO_3H_2$)-Leu-OH, trifluoroacétate**

avec pour $X_2$ : $R_5$ = OH et $R_5$ = H

**<u>EXEMPLE 26</u>** : **$X_3$-Gly-Asp-Phe-Glu-Abo-Ile-Pro-Glu-Glu-Tyr($mPO_3H_2$)-Leu-glu-OH, trifluoroacétate**

avec pour $X_3$ : n = n' = 2

**<u>EXEMPLE 27</u>** : **G-Gly-Asp-Phe-Glu-Abo-Ile-Abo-Glu-Glu-Tyr($mPO_3H_2$)-Leu-βAla-OH, trifluoroacétate**

**<u>EXEMPLE 28</u> : G-Gly-Asp-Phe-Glu-Abo-Ile-Pip-Glu-Glu-Tyr($mPO_3H_2$)-Leu-4Abu-OH, trifluoroacétate**

**<u>EXEMPLE 29</u>** : **G-Phe-Glu-Abo-Ile-Pro-Glu-Glu-Tyr($mPO_3H_2$)-Leu-glu-OH, trifluoroacétate**

**<u>EXEMPLE 30</u> : G-Glu-Abo-Ile-Pro-Glu-Glu-Tyr($mPO_3H_2$)-Leu-glu-OH, trifluoroacétate**

**<u>EXEMPLE 31</u>** : **G-Phe-Glu-Abo-Ile-Pro-Glu-Glu-Tyr($pPO_3H_2$)-Leu-βAla-OH, trifluoroacétate**

**<u>EXEMPLE 32</u>** : **G-Phe-Glu-Abo-Ile-Pro-Glu-Glu-Phe($pPO_3H_2$)-Leu-glu-OH, trifluoroacétate**

**<u>EXEMPLE 33</u>** : **G-Acc-Asp-Phe-Glu-Abo-Ile-Pro-Glu-Glu-Phe($pCH_2CO_2H$)-Leu-glu-OH, trifluoroacétate**

**<u>EXEMPLE 34</u> : G-Gly-Asp-Phe-Glu-Abo-Ile-Pro-Glu-Glu-Phe($pCH_2CO_2H$)-Leu-glu-OH, trifluoroacétate**

**<u>EXEMPLE 35</u> : G-Gly-Asp-Phe-Glu-Abo-Ile-Pro-Glu-Glu-phe($pCH_2CO_2H$)-Leu-glu-OH, trifluoroacétate**

**<u>EXEMPLE 36</u> : G-Acc-Asp-Phe-Glu-Abo-Ile-Oic-Glu-Glu-Phe($pCH_2CO_2H$)-Leu-glu-OH, trifluoroacétate**

**<u>EXEMPLE 37</u> : G-Gly-Asp-Phe-Glu-Abo-Ile-Oic-Glu-Glu-Phe($pCH_2CO_2H$)-Leu-glu-OH, trifluoroacétate**

**<u>EXEMPLE 38</u> : G-Gly-Asp-Phe-Glu-Abo-Ile-Pip-Glu-Glu-Phe($pCH_2CO_2H$)-Leu-glu-OH, trifluoroacétate**

**<u>EXEMPLE 39</u>** : **$X_0$-Acc-Asp-Phe-Glu-Abo-Ile-Oic-Glu-Glu-Phe($pCH_2CO_2H$)-Leu-glu-OH, trifluoroacétate**

avec pour $X_0$ : p = 1

**EXEMPLE 40** : **$X_0$-Acc-Asp-Phe-Glu-Abo-Ile-Pip-Glu-Glu-Phe($pCH_2CO_2H$)-Leu-glu-OH, trifluoroacétate**

avec pour $X_0$ : p = 1

**EXEMPLE 41** : **$X_2$-Acc-Asp-Phe-Glu-Abo-Ile-Abo-Glu-Glu-Phe($pCH_2CO_2H$)-Leu-glu-OH, trifluoroacétate**

avec pour $X_2$ : $R_5$ = OH, $R_5$ = H

**EXEMPLE 42** : **$X_3$-Acc-Asp-Phe-Glu-Abo-Ile-Abo-Glu-Glu-Phe($pCH_2CO_2H$)-Leu-glu-OH, trifluoroacétate**

**avec pour $X_3$: n = n' = 2**

**EXEMPLE 43** : **$X_3$-Acc-Asp-Phe-Glu-Abo-Ile-Pip-Glu-Glu-Phe($pCH_2CO_2H$)-Leu-glu-OH, trifluoroacétate**

avec pour $X_3$ : n = 2, n' = 1

**EXEMPLE 44** : **G-Acc-Asp-Phe-Glu-Abo-Ile-Pip-Glu-Glu-Phe($pCH_2CO_2H$)-Leu-glu-($CH_2OH$), trifluoroacétate**

Le $CH_2OH$ signifiant que le groupement carboxyle terminal est réduit en alcool -$CH_2OH$.

ETUDE PHARMACOLOGIQUE DES COMPOSES DE L'INVENTION

**EXEMPLE 45 : Activité antithrombotique**

Le modèle de thrombose expérimentale utilisé est celui d'une thrombose veineuse induite chez le rat Wistar mâle par ligature de la veine cave inférieure sous la veine rénale gauche.

Les animaux à tester sont anesthésiés par administration intrapéritonéale de Brietal® (méthohexital sodique) à la dose de 1 ml/kg.

La ligature est alors réalisée. Le produit à tester est injecté 2 heures plus tard par voie sous cutanée dans la région sous clavière gauche. 4 heures après cette injection, le sang est prélevé par voie intracardiaque directe, la veine est excisée et le caillot est récupéré puis pesé après passage dans une étuve à 37°C pendant 12 heures.

Les composés de l'invention ont été testés à une dose unique de 250 µg/kg par rapport à de l'hirudine non sulfatée utilisée comme composé témoin.

Les résultats de ce test ont montré que les composés de l'invention permettent une diminution du poids du caillot significativement plus importante que celle observée lors de l'injection d'hirudine non sulfatée.

**EXEMPLE 46 : Activité anticoagulante, mesure du temps de thrombine**

En présence d'une quantité standard de thrombine, un plasma normal coagule en un temps défini et constant, appelé temps de thrombine (TT). Tout allongement de ce temps traduit une anomalie de la fibrinoformation (coagulation).

Les rats Sprague-Dawley sont anesthésiés avec du pentobarbital sodique (60 mg/kg, i.p.), l'artère carotide est cathétérisée et les prélèvements sanguins réalisés dans une solution de citrate trisodique (0.109 M). Un plasma pauvre en plaquettes est obtenu par centrifugation des échantillons sanguins (3000 g, 15 min.). Le plasma peut être conservé 8 heures à 20°C.

Le temps de thrombine est réalisé avec le réactif thrombine Prest et déterminé automatiquement en utilisant un coagulomètre.

L'antagoniste ou le solvant (10 µl) est additionné au plasma (90 µl), puis incubé 2 minutes à 37°C. 100 µl de thrombine sont ajoutés en déclenchant le chronomètre.

Dans nos conditions, les TT obtenus dans le plasma témoin sont de l'ordre de 30 secondes. L'activité d'un antagoniste est évaluée par sa capacité à prolonger ce temps de thrombine par rapport au témoin.

Dans ces conditions, les composés de l'invention permettent une prolongation du temps de thrombine de 50 fois et plus. Cette prolongation est au minimum 30 fois supérieure à celle obtenue avec l'hirudine 55-65 non sulfatée. Ces résultats sont nettement meilleurs que ceux obtenus avec les dérivés de l'art antérieur.

**EXEMPLE 47 : Agrégation plaquettaire**

Dans ce test, l'activité antithrombine des antagonistes est évaluée en mesurant l'inhibition de l'agrégation plaquettaire induite par la thrombine.

Les lapins (2-3 kg) sont anesthésiés avec du sodium pentobarbital (30 mg/kg, i.v.). Après canulation de l'artère carotide gauche, le sang est prélevé sur citrate de sodium (0.109 M) (1 vol de citrate pour 9 vol de sang).

Le plasma riche en plaquettes (PRP) est obtenu par centrifugation (20°C) à 250 g pendant 20 minutes et le plasma pauvre en plaquettes (PPP) par centrifugation à 1000 g pendant 10 minutes. Le nombre des plaquettes est ajusté entre 300 000-350 000 pl/mm3 par dilution en PPP autologue. Le PRP est conservé à la température de la pièce jusqu'au moment du test et est utilisé dans les 4 heures qui suivent le prélèvement.

Le PRP est de nouveau centrifugé (900 g, 15 minutes) et les plaquettes sont lavées dans une solution saline tamponnée avec du Tris, contenant de la gélatine (0.2 %). Les plaquettes lavées (PL) sont ensuite resuspendues dans une solution physiologique et conservées à la température de la pièce.

L'agrégation plaquettaire est réalisée à 37°C dans des tubes en verre siliconés à l'aide d'un agrégomètre. Le PRP et les PL sont agités à 1000 rpm (révolutions par minute).

La thrombine est incubée 3 minutes à 37°C avec la substance à tester (à différentes concentrations) ou avec le solvant dans un volume de 50 µl. Les PL (225 µl) sont préincubées 1 minute à 37°C dans une cuvette en verre siliconée sous agitation (1000 rpm). L'agrégation est obtenue par addition de 25 µl de la solution contenant la thrombine et la substance à tester. La réponse agrégante est enregistrée pendant au moins 5 minutes.

L'intensité de l'agrégation plaquettaire est établie en prenant l'amplitude maximale des tracés agrégants et est exprimée en pourcentage de transmission lumineuse (%T). Pour chacun des composés testés, une $IC_{50}$ a été déterminée.

Les composés de l'invention inhibent de façon importante l'agrégation plaquettaire induite par la thrombine.

**EXEMPLE 48 : Mesure de l'activité anticoagulante ex vivo. Temps de prothrombine**

Les rats OFA, à jeun ou pas, sont anesthésiés au pentobarbital (60 mg/kg, i.p.). La carotide et la jugulaire sont dégagées et catétherisées. Les catéthers sont purgés avec du sérum physiologique citraté (1/40). Après installation des catéthers, un prélèvement de 1,5 cm3 de sang artériel est effectué sur citrate 0.109 M (1/9).

30 minutes après, le produit à tester est administré sous un volume de 1 ml en i.v.

Des prélèvements artériels (1,5 ml) sont alors effectués à 1 min 30, 3 min, 5 min, 15 min, 30 min et 60 min.

A chaque prélèvement 1,5 ml de serum physiologique citraté est réinjecté à l'animal via la carotide.

Les tubes de sang sont centrifugés 15 min à 4000 rpm (préparation de plasma).

100 µl de plasma sont incubés avec 200 µl de néoplastine calcique. Le temps d'apparition du phénomène de coagulation est mesuré.

Les composés de l'invention, testés à des doses de 4 à 16 mg/kg, augmentent de façon dose dépendante le temps de prothrombine (TP). Leur effet est plus important que celui de l'hirudine non sulfatée. En effet, ils augmentent le TP de façon significative. D'autre part, alors que l'activité in vivo de l'hirudine est de courte durée (30 minutes), l'augmentation du TP notée avec les composés de l'invention est toujours significative 60 minutes après leur administration.

**EXEMPLE 49 : Compositions pharmaceutiques**

Soluté injectable :

G-Gly-Asp-Phe-Glu-Abo-Ile-Pro-Glu-Glu-Tyr($mPO_3H_2$)-Leu-glu-OH : 5 mg
Eau distillée pour préparation injectable, QSP : 25 ml

Comprimé :

Formule de préparation pour 1000 comprimés

| | |
|---|---|
| G-Gly-Asp-Phe-Glu-Abo-Ile-Pro-Glu-Glu-Tyr($mPO_3H_2$)-Leu-glu-OH . . . | 5 g |
| Amidon de blé . . . . . . . . . . . . . . . . . . . . . . . . . | 10 g |
| Amidon de maïs . . . . . . . . . . . . . . . . . . . . . . . . . | 10 g |
| Lactose . . . . . . . . . . . . . . . . . . . . . . . . . . . . | 60 g |
| Stéarate de magnésium . . . . . . . . . . . . . . . . . . . . . . | 2 g |
| Hydroxypropylcellulose . . . . . . . . . . . . . . . . . . . . . | 2 g |

Une enveloppe supplémentaire assurera la gastrorésistance de la forme galénique.

Seq ID N° 1 : G-Gly-Asp-Phe-Glu-Abo-Ile-Pro-Glu-Glu-Tyr(m$PO_3H_2$)-Leu-glu-OH, trifluoroacetate

Seq ID N° 2 : G-Acc-Asp-Phe-Glu-Abo-Ile-Pro-Glu-Glu-Tyr(p$PO_3H_2$)-Leu-glu-OH, trifluoroacetate

Seq ID N° 3 : G-Gly-Asp-Phe-Glu-Abo-Ile-Pro-Glu-Glu-Tyr(p$PO_3H_2$)-Leu-glu-OH, trifluoroacetate

Seq ID N° 4 : G-Acc-Asp-Phe-Glu-Abo-Ile-Pip-Glu-Glu-Tyr(m$PO_3H_2$)-Leu-glu-OH, trifluoroacetate

Seq ID N° 5 : G-Acc-Asp-Phe-Glu-Abo-Ile-Pip-Glu-Glu-Tyr(p$PO_3H_2$)-Leu-glu-OH, trifluoroacetate

Seq ID N° 6 : G-Gly-Asp-Tyr(p$PO_3H_2$)-Glu-Abo-Ile-Pro-Glu-Glu-Tyr(p$PO_3H_2$)-Leu-glu-OH, trifluoroacetate

Seq ID N° 7 : G-Gly-Asp-Phe-Glu-Abo-Ile-Oic-Glu-Glu-Tyr(p$PO_3H_2$)-Leu-glu-OH, trifluoroacetate

Seq ID N° 8 : G-Acc-Asp-Phe-Glu-Abo-Ile-Olc-Glu-Glu-Tyr(p$PO_3H_2$)-Leu-glu-OH, trifluoroacetate

Seq ID N° 9 : G-Acc-Asp-Tyr(p$PO_3H_2$)-Glu-Abo-Ile-Pro-Glu-Glu-Tyr(p$PO_3H_2$)-Leu-glu-OH, trifluoroacetate

Seq ID N° 10 : G-Acc-Asp-Tyr(p$PO_3H_2$)-Glu-Abo-Ile-Oic-Glu-Glu-Tyr(p$PO_3H_2$)-Leu-glu-OH, trifluoroacetate

Seq ID N° 11 : G-Gly-Asp-Tyr(p$PO_3H_2$)-Glu-Abo-Ile-Oic-Glu-Glu-Tyr(p$PO_3H_2$)-Leu-glu-OH, trifluoroacetate

Seq ID N° 12 : G-Gly-Asp-Tyr(p$PO_3H_2$)-Glu-Abo-Ile-Pro-Glu-Glu-Tyr(m$PO_3H_2$)-Leu-glu-OH, trifluoroacetate

Seq ID N° 13 : G-Gly-Asp-Tyr(p$PO_3H_2$)-Glu-Abo-Ile-Oic-Glu-Glu-Tyr(m$PO_3H_2$)-Leu-glu-OH, trifluoroacetate

Seq ID N° 14 : G-Acc-Asp-Tyr(p$PO_3H_2$)-Glu-Abo-Ile-Pro-Glu-Glu-Tyr(m$PO_3H_2$)-Leu-glu-OH, trifluoroacetate

Seq ID N° 15 : G-Acc-Asp-Phe-Glu-Abo-Ile-Pro-Glu-Glu-Phe(p$PO_3H_2$)-Leu-glu-OH, trifluoroacetate

Seq ID N° 16 : G-Gly-Asp-Phe-Glu-Abo-Ile-Oic-Glu-Glu-Phe(p$PO_3H_2$)-Leu-glu-OH, trifluoroacetate

Seq ID N° 17 : G-Gly-Asp-Phe-Glu-Abo-Ile-Pro-Glu-Glu-Phe(p$PO_3H_2$)-Leu-glu-OH, trifluoroacetate

Seq ID N° 18 : G-Acc-Asp-Phe-Glu-Abo-Ile-Oic-Glu-Glu-Phe(p$PO_3H_2$)-Leu-glu-OH, trifluoroacetate

Seq ID N° 19 : G-Acc-Asp-Phe(p$PO_3H_2$)-Glu-Abo-Ile-Pro-Glu-Glu-Phe(p$PO_3H_2$)-Leu-glu-OH, trifluoroacetate

Seq ID N° 20 : G-Acc-Asp-Phe(p$PO_3H_2$)-Glu-Abo-Ile-Oic-Glu-Glu-Phe(p$PO_3H_2$)-Leu-glu-OH, trifluoroacetate

Seq ID N° 21 : G-Gly-Asp-Phe(p$PO_3H_2$)-Glu-Abo-Ile-Oic-Glu-Glu-Phe(p$PO_3H_2$)-Leu-glu-OH, trifluoroacetate

Seq ID N° 22 : $X_0$-Gly-Asp-Tyr-Glu-Abo-Ile-Pro-Glu-Glu-Tyr(p$PO_3H_2$)-Leu-glu-OH, trifluoroacetate

Seq ID N° 23 : $X_0$-Gly-Asp-Tyr-Glu-Abo-Ile-Oic-Glu-Glu-Tyr(p$PO_3H_2$)-Leu-glu-OH, trifluoroacetate

Seq ID N° 24 : $X_0$-Acc-Asp-Tyr-Glu-Abo-Ile-Pro-Glu-Glu-Tyr(p$PO_3H_2$)-Leu-glu-OH, trifluoroacetate

Seq ID N° 25 : $X_2$-Gly-Asp-Phe-Glu-Abo-Ile-Pip-Glu-Glu-Tyr(m$PO_3H_2$)-Leu-OH, trifluoroacetate

Seq ID N° 26 : $X_3$-Gly-Asp-Phe-Glu-Abo-Ile-Pro-Glu-Glu-Tyr(m$PO_3H_2$)-Leu-glu-OH, trifluoroacetate

Seq ID N° 27 : G-Gly-Asp-Phe-Glu-Abo-Ile-Abo-Glu-Glu-Tyr($mPO_3H_2$)-Leu-βAla-OH, trifluoroacetate

Seq ID N° 28 : G-Gly-Asp-Phe-Glu-Abo-Ile-Pip-Glu-Glu-Tyr($mPO_3H_2$)-Leu-4Abu-OH, trifluoroacetate

Seq ID N° 29 : G-Phe-Glu-Abo-Ile-Pro-Glu-Glu-Tyr($mPO_3H_2$)-Leu-glu-OH, trifluoroacetate

Seq ID N° 30 : G-Glu-Abo-Ile-Pro-Glu-Glu-Tyr($mPO_3H_2$)-Leu-glu-OH, trifluoroacetate

Seq ID N° 31 : G-Phe-Glu-Abo-Ile-Pro-Glu-Glu-Tyr($pPO_3H_2$)-Leu-βAla-OH, trifluoroacetate

Seq ID N° 32 : G-Phe-Glu-Abo-Ile-Pro-Glu-Glu-Phe($pPO_3H_2$)-Leu-glu-OH, trifluoroacetate

Seq ID N° 33 : G-Acc-Asp-Phe-Glu-Abo-Ile-Pro-Glu-Glu-Phe($pCH_2CO_2H$)-Leu-glu-OH, trifluoroacetate

Seq ID N° 34 : G-Gly-Asp-Phe-Glu-Abo-Ile-Pro-Glu-Glu-Phe($pCH_2CO_2H$)-Leu-glu-OH, trifluoroacetate

Seq ID N° 35 : G-Gly-Asp-Phe-Glu-Abo-Ile-Pro-Glu-Glu-phe($pCH_2CO_2H$)-Leu-glu-OH, trifluoroacetate

Seq ID N° 36 : G-Acc-Asp-Phe-Glu-Abo-Ile-Oic-Glu-Glu-Phe($pCH_2CO_2H$)-Leu-glu-OH, trifluoroacetate

Seq ID N° 37 : G-Gly-Asp-Phe-Glu-Abo-Ile-Oic-Glu-Glu-Phe($pCH_2CO_2H$)-Leu-glu-OH, trifluoroacetate

Seq ID N° 38 : G-Gly-Asp-Phe-Glu-Abo-Ile-Pip-Glu-Glu-Phe($pCH_2CO_2H$)-Leu-glu-OH, trifluoroacetate

Seq ID N° 39 : $X_0$-Acc-Asp-Phe-Glu-Abo-Ile-Oic-Glu-Glu-Phe($pCH_2CO_2H$)-Leu-glu-OH, trifluoroacetate

Seq ID N° 40 : $X_0$-Acc-Asp-Phe-Glu-Abo-Ile-Pip-Glu-Glu-Phe($pCH_2CO_2H$)-Leu-glu-OH, trifluoroacetate

Seq ID N° 41 : $X_2$-Acc-Asp-Phe-Glu-Abo-Ile-Abo-Glu-Glu-Phe($pCH_2CO_2H$)-Leu-glu-OH, trifluoroacetate

Seq ID N° 42 : $X_3$-Acc-Asp-Phe-Glu-Abo-Ile-Abo-Glu-Glu-Phe($pCH_2CO_2H$)-Leu-glu-OH, trifluoroacetate

Seq ID N° 43 : $X_3$-Acc-Asp-Phe-Glu-Abo-Ile-Pip-Glu-Glu-Phe($pCH_2CO_2H$)-Leu-glu-OH, trifluoroacetate

Seq ID N° 44 : G-Acc-Asp-Phe-Glu-Abo-Ile-Pip-Glu-Glu-Phe($pCH_2CO_2H$)-Leu-glu-($CH_2OH$), trifluoroacetate

## Revendications

**1/** Composés de formule générale (I) :

$$X - A_1 - A_2 - A_3 - A_4 - A_5 - A_6 - A_7 - A_8 - A_9 - A_{10} - Y \qquad (I)$$

dans laquelle :

X représente le substituant du groupement aminé terminal du peptide de formule (I) et représente un groupement guanidinoacyl ($C_1$-$C_6$) linéaire ou ramifié choisi parmi les groupements

$X_0$ = 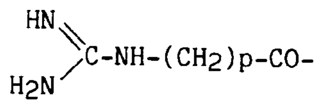 ,

avec $1 \leqq p \leqq 5$ ,

un groupement guanidinoalkyl ($C_1$-$C_6$) linéaire ou ramifié ou l'un quelconque des groupements suivants :

$X_1$ = 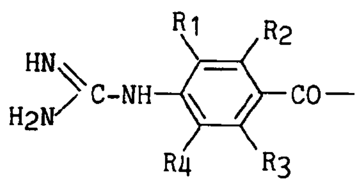

avec $R_1$, $R_2$, $R_3$, $R_4$ identiques ou différents représentant indépendamment l'un de l'autre un atome d'hydrogène, un groupement alkyle ($C_1$-$C_6$) linéaire ou ramifié, un atome d'halogène, un groupement trifluorométhyle, un groupement hydroxy, un groupement alkoxy ($C_1$-$C_6$) linéaire ou ramifié, un groupement thio, un groupement alkylthio ($C_1$-$C_6$) linéaire ou ramifié, un groupement cyano, un groupement nitro ou un groupement phényloxy,

$X_2$ = 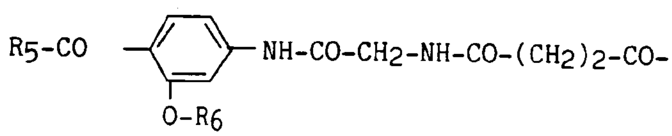 ,

où $R_5$ représente un groupement alkoxy inférieur ($C_1$-$C_6$) ou un groupement hydroxyle et où $R_5$ représente un atome d'hydrogène ou un groupement acyle inférieur ($C_1$-$C_6$),

ou

$X_3$ = 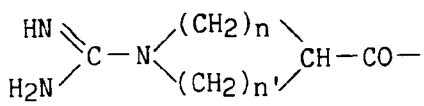 ,

avec n et n' entiers compris entre 1 et 6,

$A_1$ représente une liaison ou un résidu peptidique contenant de 1 à 3 amino-acides quelconques, parmi lesquels peut figurer un résidu $A_{11}$ de formule :

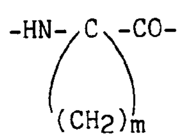

(avec m entier compris entre 2 et 7)

$A_2$ représente une liaison ou un résidu phénylalanine (Phe), tyrosine (Tyr), isoleucine (Ile), norvaline (Nva), (1,2,3,4)-tétrahydro-isoquinoline-3-carbonyl (Tic), un résidu acide α-aminobutyrique (Abu), acide glutamique (Glu), ou un résidu d'acide aminé contenant un groupement aryle, un résidu $A_{11}$ ou un résidu $A_9$,

$A_3$ représente une liaison ou un résidu acide glutamique (Glu), acide aspartique (Asp), β-alanine (βAla), tyrosine (Tyr) ou un résidu $A_{11}$,

$A_4$ représente une liaison ou un résidu acide glutamique (Glu), acide aspartique (Asp), proline (Pro), acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh) ou un résidu $A_{11}$,

$A_5$ représente une liaison ou un résidu isoleucine (Ile), norvaline (Nva), phénylalanine (Phe), proline (Pro), ornithine (Orn), acide 2,3-diaminopropionique (Dpr) ou un résidu $A_{11}$,

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro), un résidu $A_{11}$ ou l'acide pipécolique (Pip),

$A_7$ représente un résidu acide glutamique (Glu), acide aspartique (Asp) ou un résidu $A_{11}$,

$A_5$ représente un résidu acide glutamique (Glu), acide aspartique (Asp), acide 2,3-diaminopropionique (Dpr), acide 2,4-diaminobutyrique (Dab), β-alanine (βAla) ou un résidu $A_{11}$,

$A_9$ représente un résidu

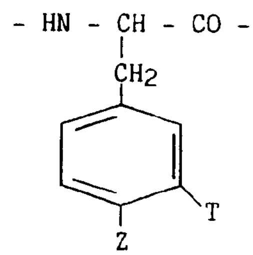

avec Z ou T, différents, représentant un atome d'hydrogène ou un groupement $OPO_3H_2$, $PO_3H_2$, $CH_2CO_2H$ ou OH à la condition que lorsque Z représente OH, T soit différent d'un atome d'hydrogène,

$A_{10}$ représente une liaison, un résidu leucine (Leu), valine (Val), β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), un résidu tripeptidique tel que Nal-Nal-Leu ou un résidu $A_{11}$ ou un fragment peptidique contenant de 1 à 3 résidus de tout acide aminé,

Y représente le substituant du groupement carbonyl terminal du peptide de formule (I) et représente un groupement hydroxy, un groupement alkoxy ($C_1$-$C_6$) linéaire ou ramifié ou un groupement amino lui-même éventuellement substitué par un ou deux groupements alkyles ($C_1$-$C_6$) linéaires ou ramifiés,
ou bien
le groupement carboxy terminal du peptide de formule (I) est réduit en alcool correspondant (ol), (groupement $-CH_2OH$),

étant entendu que par groupement aryle on entend phényle ou naphtyle,

le composé de formule I pouvant éventuellement comprendre :

- une liaison pseudopeptidique telle que $-CH_2-NH-$, $-CH_2-S-$, $-CH_2-SO-$, $-CH_2-SO_2-$, -NH-CO-, -CH=CH- remplaçant une liaison peptidique -CO-NH-,
  et/ou
- une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,

leurs sels d'addition à un acide ou à une base pharmaceutiquement acceptable, chaque amino-acide de la séquence peptidique étant optiquement pur et le carbone α de chaque amino-acide ayant la configuration D ou L.

**2/** Composés selon la revendication 1 tels que X représente un groupement $X_1$ pour lequel $R_1$, $R_2$, $R_3$, $R_4$ représentent simultanément un atome d'hydrogène, de formule :

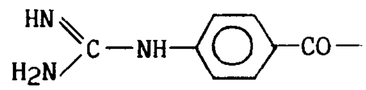

ainsi que leurs sels d'addition à un acide ou une base pharmaceutiquement acceptable, chaque amino-acide de la séquence peptidique étant optiquement pur et le carbone α de chaque amino-acide ayant la configuration D ou L.

**3/** Composés selon la revendication 1 dans lesquels le résidu $A_9$ est tel que Z représente un groupement $OPO_3H_2$ et T un atome d'hydrogène,
ainsi que leurs sels d'addition à un acide ou une base pharmaceutiquement acceptable, chaque amino-acide de la séquence peptidique étant optiquement pur et le carbone α de chaque amino-acide ayant la configuration D ou L.

**4/** Composés selon la revendication 1 dans lesquels le résidu $A_9$ est tel que Z représente un groupement hydroxy et T un groupement $PO_3H_2$, ainsi que leurs sels d'addition à un acide ou une base pharmaceutiquement acceptables, chaque amino-acide de la séquence peptidique étant optiquement pur et le carbone α de chaque amino-acide ayant la configuration D ou L.

**5/** Composés selon la revendication 1 dans lesquels le résidu $A_9$ est tel que Z représente un groupement $PO_3H_2$ et T un atome d'hydrogène, leurs sels d'addition à un acide ou une base pharmaceutiquement acceptable, chaque amino-acide de la séquence peptidique étant optiquement pur et le carbone α de chaque amino-acide ayant la configuration D ou L.

**6/** Composés selon la revendication 1 dans lesquels le résidu $A_9$ est tel que Z représente un groupement $CH_2CO_2H$ et T un atome d'hydrogène, leurs sels d'addition à un acide ou une base pharmaceutiquement acceptable, chaque amino-acide de la séquence peptidique étant optiquement pur et le carbone α de chaque amino-acide ayant la configuration D ou L.

**7/** Composé selon la revendication 1 qui est le G-Gly-Asp-Phe-Glu-Abo-Ile-Pro-Glu-Glu-Tyr($mPO_3H_2$)-Leu-glu-OH, où G représente le radical :

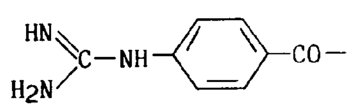

et Tyr($mPO_3H_2$) le résidu:

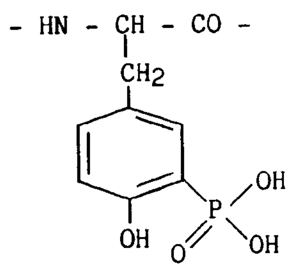

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**8/** Composé selon la revendication 1 qui est le G-Gly-Asp-Phe-Glu-Abo-Ile-Pro-Glu-Glu-Tyr($pPO_3H_2$)-Leu-glu-OH, où G représente le radical :

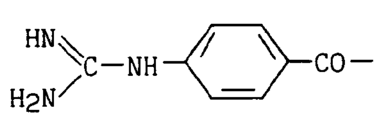

et Tyr($pPO_3H_2$) le résidu:

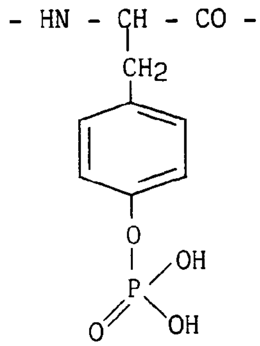

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**9/** Composé selon la revendication 1 qui est le G-Gly-Asp-Phe-Glu-Abo-Ile-Pro-Glu-Glu-Phe($pPO_3H_2$)-Leu-glu-OH, où G représente le radical :

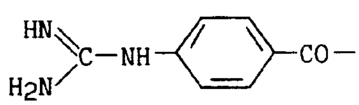

et Phe($pPO_3H_2$) représente :

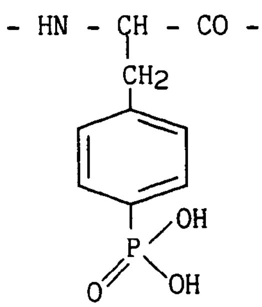

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**10/** Composé selon la revendication 1 qui est le G-Gly-Asp-Phe-Glu-Abo-Ile-Pro-Glu-Glu-Phe($pCH_2CO_2H$)-Leu-glu-OH, où G représente le radical :

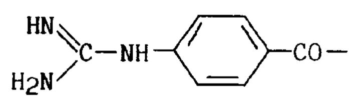

et Phe($pCH_2CO_2H$) représente :

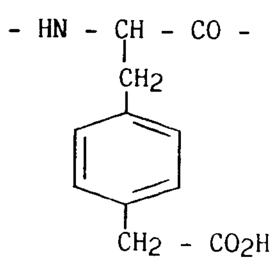

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**11/** Procédé de préparation des composés de formule (I) selon la revendication 1 caractérisé en ce qu'ils peuvent être obtenus par synthèse séquentielle sur phase solide à partir d'amino-acides protégés, par synthèse à partir de fragments peptidiques en solution,
éventuellement par combinaison de ces deux techniques,
si on le désire par cyclisation totale ou partielle à travers deux fonctions déprotégées de façon sélective et capable de former une liaison covalente,
et si on le souhaite par introduction,
selon les techniques classiques de la chimie organique, d'une liaison pseudopeptidique à tout moment de synthèse de la séquence peptidique,
dérivés de formule (I) que l'on transforme, si nécessaire, en leurs sels d'addition à un acide ou à une base pharmaceutiquement acceptable.

**12/** Compositions pharmaceutiques contenant comme principe actif au moins un composé selon l'une quelconque des revendications 1 à 10, seul ou en combinaison avec un ou plusieurs excipients ou véhicules inertes, non toxiques, pharmaceutiquement acceptables.

**13/** Compositions pharmaceutiques selon la revendication 12 contenant au moins un principe actif selon l'une quelconque des revendications 1 à 10 utiles comme anticoagulants, dans le traitement des thromboses veineuses aigües, de l'embolie pulmonaire, dans l'embolie artérielle des extrêmités, de l'infarctus du myocarde, de l'athérosclérose et des manifestations thromboemboliques, ainsi que pour maintenir l'homéostasie sanguine en particulier dans la circulation extracorporelle.

**Revendications pour l'Etat contractant suivant : ES**

**1/** Procédé de préparation des composés de formule générale (I) :

$$X - A_1 - A_2 - A_3 - A_4 - A_5 - A_6 - A_7 - A_8 - A_9 - A_{10} - Y \qquad (I)$$

dans laquelle :

X représente le substituant du groupement aminé terminal du peptide de formule (I) et représente un groupement guanidinoacyl ($C_1$-$C_6$) linéaire ou ramifié choisi parmi les groupements

$X_0$ = 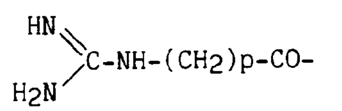 ,

avec $1 \leqq p \leqq 5$ ,
un groupement guanidinoalkyl ($C_1$-$C_6$) linéaire ou ramifié ou l'un quelconque des groupements suivants :

$X_1$ = 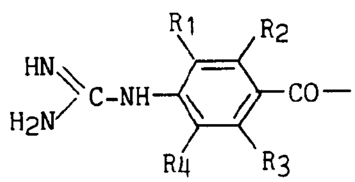

avec $R_1$, $R_2$, $R_3$, $R_4$ identiques ou différents représentant indépendamment l'un de l'autre un atome d'hydrogène, un groupement alkyle ($C_1$-$C_6$) linéaire ou ramifié, un atome d'halogène, un groupement trifluorométhyle, un groupement hydroxy, un groupement alkoxy ($C_1$-$C_6$) linéaire ou ramifié, un groupement thio, un groupement alkylthio ($C_1$-$C_6$) linéaire ou ramifié, un groupement cyano, un groupement nitro ou un groupement phényloxy,

$X_2$ = 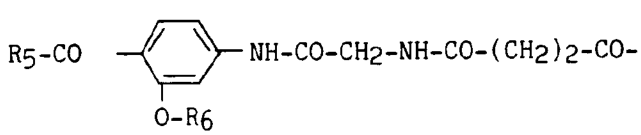 ,

où $R_5$ représente un groupement alkoxy inférieur ($C_1$-$C_6$) ou un groupement hydroxyle et où $R_5$ représente un atome d'hydrogène ou un groupement acyle inférieur ($C_1$-$C_6$), ou

$X_3$ = 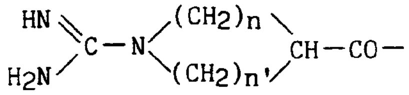 ,

avec n et n' entiers compris entre 1 et 6,

$A_1$ représente une liaison ou un résidu peptidique contenant de 1 à 3 amino-acides quelconques, parmi lesquels peut figurer un résidu $A_{11}$ de formule :

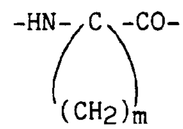

(avec m entier compris entre 2 et 7)

$A_2$ représente une liaison ou un résidu phénylalanine (Phe), tyrosine (Tyr), isoleucine (Ile), norvaline (Nva), (1,2,3,4)-tétrahydro-isoquinoline-3-carbonyl (Tic), un résidu acide α-aminobutyrique (Abu), acide glutamique (Glu), ou un résidu d'acide aminé contenant un groupement aryle, un résidu $A_{11}$ ou un résidu $A_9$,

$A_3$ représente une liaison ou un résidu acide glutamique (Glu), acide aspartique (Asp), β-alanine (βAla), tyrosine (Tyr) ou un résidu $A_{11}$,

$A_4$ représente une liaison ou un résidu acide glutamique (Glu), acide aspartique (Asp), proline (Pro), acide 2,3-diaminopropionique (Dpr), acide 2,4-diaminobutyrique (Dab), ornithine (Orn), lysine (Lys), 2-azabicyclo [2.2.2] octane-3-carbonyl (Abo), 2-azabicyclo [2.2.1] heptane-3-carbonyl (Abh) ou un résidu $A_{11}$,

$A_5$ représente une liaison ou un résidu isoleucine (Ile), norvaline (Nva), phénylalanine (Phe), proline (Pro), ornithine (Orn), acide 2,3-diaminopropionique (Dpr) ou un résidu $A_{11}$,

$A_5$ représente un résidu proline (Pro), isoleucine (Ile), norvaline (Nva), phénylalanine (Phe), ornithine (Orn), 2-azabicyclo [2.2.2] octane-3-carbonyl (Abo), 2-azabicyclo [2.2.1] heptane-3-carbonyl (Abh), octahydroindole-2-carbonyl (Oic) ou 3,4-déhydroproline (dhPro), un résidu $A_{11}$ ou l'acide pipécolique (Pip),

$A_7$ représente un résidu acide glutamique (Glu), acide aspartique (Asp) ou un résidu $A_{11}$,

$A_5$ représente un résidu acide glutamique (Glu), acide aspartique (Asp), acide 2,3-diaminopropionique (Dpr), acide 2,4-diaminobutyrique (Dab), β-alanine (βAla) ou un résidu $A_{11}$,

$A_9$ représente un résidu

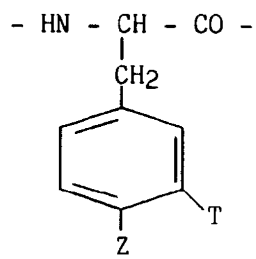

avec Z ou T, différents, représentant un atome d'hydrogène ou un groupement $OPO_3H_2$, $PO_3H_2$, $CH_2CO_2H$ ou OH à la condition que lorsque Z représente OH, T soit différent d'un atome d'hydrogène,

$A_{10}$ représente une liaison, un résidu leucine (Leu), valine (Val), β-naphtylalanine (Nal), cyclohexylalanine (Cha), β-(2-thiényl)alanine (Thi), octahydroindole-2-carbonyl (Oic), un résidu dipeptidique tel que Leu-Glu, Leu-Pro, Leu-βAla, Val-Glu, Nal-Glu, Cha-Glu- Thi-Glu, Oic-Glu, leucine-acide 4-aminobutyrique (Leu-4Abu), un résidu tripeptidique tel que Nal-Nal-Leu ou un résidu $A_{11}$ ou un fragment peptidique contenant de 1 à 3 résidus de tout acide aminé,

Y représente le substituant du groupement carbonyl terminal du peptide de formule (I) et représente un groupement hydroxy, un groupement alkoxy ($C_1$-$C_6$) linéaire ou ramifié ou un groupement amino lui-même éventuellement substitué par un ou deux groupements alkyles ($C_1$-$C_6$) linéai-

res ou ramifiés,
ou bien
le groupement carboxy terminal du peptide de formule (I) est réduit en alcool correspondant (ol), (groupement $-CH_2OH$),

étant entendu que par groupement aryle on entend phényle ou naphtyle,
le composé de formule I pouvant éventuellement comprendre :

- une liaison pseudopeptidique telle que $-CH_2-NH-$, $-CH_2-S-$, $-CH_2-SO-$, $-CH_2-SO_2-$, -NH-CO-, -CH=CH- remplaçant une liaison peptidique -CO-NH-,
  et/ou
- une cyclisation non cystéinique entre les chaînes latérales de deux amino-acides, ou entre un groupement amino terminal et une chaîne latérale d'un amino-acide ou entre un groupement carboxy terminal et une chaîne latérale d'un amino-acide ou entre un groupement amino terminal et un groupement carboxy terminal,

leurs sels d'addition à un acide ou à une base pharmaceutiquement acceptable, chaque amino-acide de la séquence peptidique étant optiquement pur et le carbone α de chaque amino-acide ayant la configuration D ou L, caractérisé en ce qu'ils peuvent être obtenus par synthèse séquentielle sur phase solide à partir d'amino-acides protégés, par synthèse à partir de fragments peptidiques en solution,
éventuellement par combinaison de ces deux techniques,
si on le désire par cyclisation totale ou partielle à travers deux fonctions déprotégées de façon sélective et capable de former une liaison covalente,
et si on le souhaite par introduction,
selon les techniques classiques de la chimie organique, d'une liaison pseudopeptidique à tout moment de synthèse de la séquence peptidique,
dérivés de formule (I) que l'on transforme, si nécessaire, en leurs sels d'addition à un acide ou à une base pharmaceutiquement acceptable.

**2/** Procédé de préparation, selon la revendication 1, des composés tels que X représente un groupement $X_1$ pour lequel $R_1$, $R_2$, $R_3$, $R_4$ représentent simultanément un atome d'hydrogène, de formule :

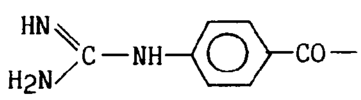

ainsi que leurs sels d'addition à un acide ou une base pharmaceutiquement acceptable, chaque amino-acide de la séquence peptidique étant optiquement pur et le carbone α de chaque amino-acide ayant la configuration D ou L.

**3/** Procédé de préparation, selon la revendication 1, des composés dans lesquels le résidu $A_9$ est tel que Z représente un groupement $OPO_3H_2$ et T un atome d'hydrogène,
ainsi que leurs sels d'addition à un acide ou une base pharmaceutiquement acceptable, chaque amino-acide de la séquence peptidique étant optiquement pur et le carbone α de chaque amino-acide ayant la configuration D ou L.

**4/** Procédé de préparation, selon la revendication 1, des composés dans lesquels le résidu $A_9$ est tel que Z représente un groupement hydroxy et T un groupement $PO_3H_2$, ainsi que leurs sels d'addition à un acide ou une base pharmaceutiquement acceptables, chaque amino-acide de la séquence peptidique étant optiquement pur et le carbone α de chaque amino-acide ayant la configuration D ou L.

**5/** Procédé de préparation, selon la revendication 1, des composés dans lesquels le résidu $A_9$ est tel que Z représente un groupement $PO_3H_2$ et T un atome d'hydrogène, leurs sels d'addition à un acide ou une base pharmaceutiquement acceptable, chaque amino-acide de la séquence peptidique étant optiquement pur et le carbone α de chaque amino-acide ayant la configuration D ou L.

**6/** Procédé de préparation, selon la revendication 1, des composés dans lesquels le résidu $A_9$ est tel que Z représente un groupement $CH_2CO_2H$ et T un atome d'hydrogène, leurs sels d'addition à un acide ou une base pharmaceutiquement acceptable, chaque amino-acide de la séquence peptidique étant optiquement pur et le carbone α de chaque amino-acide ayant la configuration D ou L.

**7/** Procédé de préparation, selon la revendication 1, du composé qui est le G-Gly-Asp-Phe-Glu-Abo-Ile-Pro-Glu-Glu-Tyr($mPO_3H_2$)-Leu-glu-OH, où G représente le radical :

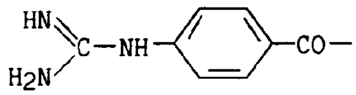

et Tyr($mPO_3H_2$) le résidu :

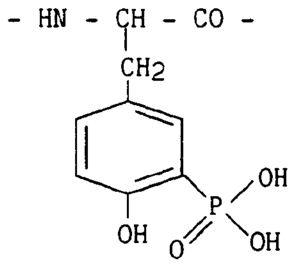

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**8/** Procédé de préparation, selon la revendication 1, du composé qui est le G-Gly-Asp-Phe-Glu-Abo-Ile-Pro-Glu-Glu-Tyr($pPO_3H_2$)-Leu-glu-OH, où G représente le radical :

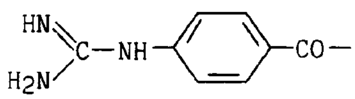

et Tyr($pPO_3H_2$) le résidu :

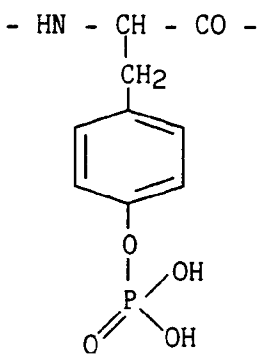

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**9/** Procédé de préparation, selon la revendication 1, du composé qui est le G-Gly-Asp-Phe-Glu-Abo-Ile-Pro-Glu-Glu-Phe($pPO_3H_2$)-Leu-glu-OH, où G représente le radical :

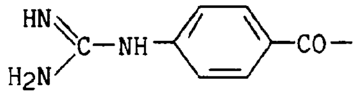

et Phe($pPO_3H_2$) représente :

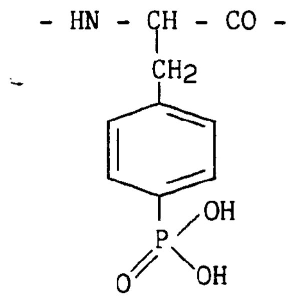

ainsi que ses sels d'addition à un acide ou une base pharmaceutiquement acceptable, chaque amino-acide de la séquence peptidique étant optiquement pur et le carbone α de chaque amino-acide ayant la configuration D ou L.

**10/** Procédé de préparation, selon la revendication 1, du composé qui est le G-Gly-Asp-Phe-Glu-Abo-Ile-Pro-Glu-Glu-Phe($pCH_2CO_2H$)-Leu-glu-OH, où G représente le radical :

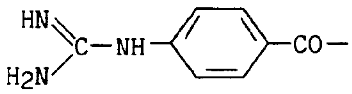

et Phe($pCH_2CO_2H$) représente :

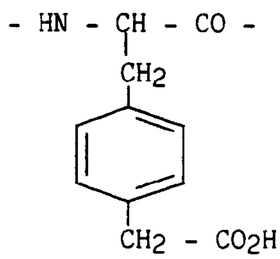

ainsi que ses sels d'addition à un acide ou une base pharmaceutiquement acceptable, chaque amino-acide de la séquence peptidique étant optiquement pur et le carbone α de chaque amino-acide ayant la configuration D ou L.

Office européen des brevets

# RAPPORT DE RECHERCHE EUROPEENNE

Numero de la demande

EP 93 40 0051
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | THROMBOSIS AND HAEMOSTASIS<br>vol. 63, no. 2, 12 Avril 1990, STUTTGART, L'ALLEMAGNE<br>pages 208 - 214<br>KRSTENANSKY ET AL 'Development of MDL 28,050, a small stable antihrombin agent based on a functionable domain of the leech protein, hirudin'<br>* En entier *<br>--- | 1-13 | C07K7/06<br>C07K7/08<br>A61K37/02 |
| A | EDITEURS: SAMMES ET AL 'Comprehensive Medicinal Chemistry; Tome 2 ( Enzymes and other molecular targets )'<br>1990 , PERGAMON PRESS , OXFORD, L'ANGLETERRE<br>* Pages 490-492, 8.4.3.5.4. Thrombin Inhibitors *<br>--- | 1-13 | |
| A | EDITEUR: MARSHALL, G.R. 'Proceedings of the 10th American Peptide Symposium, Mai 1987, St. Louis, Miss., Les États-Unis'<br>1988 , ESCOM , LEIDEN, PAYS-BAS<br>& KRSTENANSKY ET AL 'Characterization of the interaction of thrombin with the C-terminal region of the leech anticoagulant peptide; hirudin; pages 447-448'<br>--- | 1-13 | **DOMAINES TECHNIQUES RECHERCHES (Int. Cl.5 )**<br>C07K |
| A | FEBS LETTERS<br>vol. 294, 9 Décembre 1991, AMSTERDAM, PAYS-BAS<br>pages 163 - 166<br>BOURDON ET AL 'Structure-function relationships of hirulog peptide interactions with thrombin'<br>* Page 165 - GBA *<br>---<br>-/-- | 1-13 | |

Le présent rapport a été établi pour toutes les revendications

| Lieu de la recherche | Date d'achèvement de la recherche | Examinateur |
|---|---|---|
| LA HAYE | 11 MARS 1993 | KORSNER S.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen des brevets

# RAPPORT DE RECHERCHE EUROPEENNE

Numero de la demande

EP 93 40 0051
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| P,X | EP-A-0 468 448 (MERRELL DOW PHARMACEUTICALS INC.)<br>29 Janvier 1992<br>* page 2, ligne 1 - page 4, ligne 49 *<br>--- | 1,11-13 | |
| P,O,<br>A | EDITEURS: SMITH ET AL 'Proceedings of the 12th American Peptide Symposium, Juin 1991, Cambridge, Mass., Les États-Unis' 1992 , ESCOM , LEIDEN, PAYS-BAS<br>& SZEWCZUK ET AL 'Synthetic bifunctional thrombin inhibitors: Linker design; pages 806-807'<br>--- | 1-13 | |
| P,O,<br>A | EDITEURS: SMITH ET AL 'Proceedings of the 12th American Peptide Symposium, Juin 1991, Cambridge, Mass., Les États-Unis' 1992 , ESCOM , LEIDEN, PAYS-BAS<br>& DIMAIO ET AL 'Homologation of the P1' site of hirutonins:a new prototype of thrombin inhibitors; pages 814-815'<br>----- | 1-13 | DOMAINES TECHNIQUES RECHERCHES (Int. Cl.5 ) |

Le présent rapport a été établi pour toutes les revendications

| Lieu de la recherche | Date d'achèvement de la recherche | Examinateur |
|---|---|---|
| LA HAYE | 11 MARS 1993 | KORSNER S.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)